# EUROPÄISCHE PATENTSCHRIFT

(11) **EP 1 683 502 B1**
(45) Veröffentlichungstag und Bekanntmachung des Hinweises auf die Patenterteilung: **11.02.2009**
(21) Anmeldenummer: 05110932.0
(22) Anmeldetag: 18.11.2005
(51) Int. Cl.: A61F 13/02

(54) **Filmpflaster**
Film wound dressing
Pansement en pellicule

(30) Priorität: 24.01.2005 DE 102005003391
(43) Veröffentlichungstag der Anmeldung: 26.07.2006
(73) Patentinhaber: Beiersdorf Aktiengesellschaft, 20253 Hamburg (DE)
(72) Erfinder: Hilfenhaus, Peter, 20253 Hamburg (DE); Bodenschatz, Stefan, 21614, Buxtehude (DE)

(56) Entgegenhaltungen:
- EP-A- 0 254 493
- EP-A- 0 630 628
- WO-A-97/25012

## Beschreibung

Die Erfindung betrifft ein Pflaster bestehend aus einer relativ dünnen Folie, einer Klebemasse zur Befestigung auf der Haut, einem Abdeckmaterial und einem unterbrochenen Stützrahmen zur Verbesserung der Applikation.

Filmpflaster, auch als Folienpflaster oder Filmdressing bekannt, werden vornehmlich in der Wundversorgung und im medizinischen Bereich zur Befestigung von Kanülen und Katheter verwendet.

Das Applizieren der aus dünnen Folien aufgebauten Filmpflaster ist sehr schwierig, da nach Abziehen des Abdeckmaterials, das eine stützende, versteifende und damit stabilisierende Wirkung ausübt, von der Klebemasse, die dünne Folie schwierig zu handhaben ist, häufig Falten wirft und an sich selbst verklebt und das Pflaster so unbrauchbar wird. Es sind eine Vielzahl von Druckschriften bekannt, die Lösungen beschreiben, dem Folienpflaster während der Applikation zusätzlich Stabilität zu verleihen.

So ist vorgeschlagen worden, die Folie mit einem zusätzlichen Träger-, Stütz-, Verstärkungsoder Versteifungsmaterial, im nachfolgenden als Stützmaterial oder -element bezeichnet, auf der nicht mit der Klebemasse beschichteten Seite zu versehen, das nach der Applikation des Pflasters entfernt werden kann. EP-A1-473918 beschreibt ein derartiges Pflaster. Zur besseren Handhabung des Pflasters sind zusätzliche Griffleisten am Stützmaterial angebracht. In EP-A1-51935 werden Filmpflaster beschrieben, die aus einer relativ dünnen Folie, einer darauf aufgebrachten Klebmasse, einem Abdeckmaterial zum Schutz der Klebemasse und einem stabilisierenden Rahmen als Stützmaterial bestehen. Das Abdeckmaterial ist einstückig auf der Haftklebemasse aufgebracht. Der Rahmen dient zur Stabilisierung der dünnen Folie beim Applizieren auf die Haut und soll das "Mit-sich-selbst-verkleben" (Verkleben der Klebemasse mit Klebmasse oder einer anderen Pflasterkomponente) verhindern. Ungünstig bei der Applikation dieser Filmpflaster ist, dass sich beim Ablösen des Abdeckmaterials von der Klebemasse der Rahmen von der Folie lösen kann. Die Verbindung des Rahmens mit der Folie ist eine rein mechanische Haftung, die z.B. beim Extrudieren der Folie auf das Rahmenmaterial entsteht. EP-A2-690706 und US2001/0027285 beschreiben ein Filmpflaster, bei dem das beschriebene Problem der sicheren Haftung des Rahmens auf der Folie dadurch gelöst wird, dass der Rahmen durch zusätzliches Heißsiegeln an der Folie fixiert wird. Dies bedingt jedoch einen zusätzlichen Arbeitschritt, der das Pflaster unnötig verteuert und die Herstellung verkompliziert.

Aufgabe der vorliegenden Erfindung ist es daher ein Filmpflaster bereit zu stellen, dass für den Anwender einfach zu applizieren und gleichzeitig einfach und kostengünstig herzustellen ist.

Gelöst wird diese Aufgabe durch ein Filmpflaster gemäß Anspruch 1.

WO 9725012 offenbart ein Filmpflaster mit einer Folie, bestehend aus mindestens einem wasserdampfdurch- und wasserundurchlässigem Polymer, eine Klebemasse, die zumindest partiell eine Seite der Folie beschichtet, ein zumindest zweigeteiltes oder teilbares Abdeckmaterial, das auf der Klebemassen aufgebracht ist und einen Stützrahmen, der auf der der Klebemasse abgewandten Seite auf der Folie angebracht ist.

Aufgabe der vorliegenden Erfindung ist es daher, ein Filmpflaster bereit zu stellen, dass für den Anwender einfach zu applizieren und gleichzeitig einfach und kostengünstig herzustellen ist.

Es war überraschend und für den Fachmann außerordentlich erstaunlich, dass ein Filmpflaster,
umfassend
a. eine Folie von bis zu 100 µm Dicke, bestehend aus mindestens einem wasserdampfdurch- und wasserundurchlässigem Polymer,
b. eine Klebemasse, die zumindest partiell eine Seite der Folie beschichtet,
c. ein zumindest zweigeteiltes oder teilbares Abdeckmaterial, das auf der Klebemasse aufgebracht ist,
d. einen zumindest zweigeteilten Stützrahmen, der auf der der Klebemasse abgewandten Seite auf der Folie angebracht ist, und
e. ein Trenn- bzw. Überlappungsbereich des Abdeckmaterials, wobei
der Stützrahmen unterbrochen ist und den Trenn- bzw Überlappungsbereich des Abdeckmaterials frei lässt.
die gestellten Aufgaben löst.

Die wesentlichen Vorteile des vorliegenden Filmpflasters sind:
- Die Versteifungs- oder Stützelemente in Form eines unterbrochenen Stützrahmens geben dem Filmpflaster Stabilität, so dass das Applizieren ohne Probleme möglich ist und das "Mit-sich-selbst-Verkleben" verhindert wird. Somit ist das Filmpflaster für den Anwender einfach anzuwenden.
- Die erfindungsgemäße Gestaltung des Stützrahmens ermöglicht deren einfache Herstellung aus einem Stützmaterial.
- Das Filmpflaster stellt bei der Herstellung keine besonderen Anforderungen an die Haftung Folie/Stützmaterial dar. Mechanische Adhäsionskräfte reichen aus, so wie sie bei der Herstellung des Films durch Extrusion, Gießen, etc. der Folie auf dem Stützmaterial, erreicht werden. Besondere, im Stand der Technik beschriebenen Maßnahmen zur Haftungsverbesserung sind daher nicht notwendig und lassen somit eine kostengünstige und einfache Herstellung zu.
- Durch das zumindest zweiteilige oder teilbare Abdeckmaterial, das auf der Klebemasse aufgebracht ist, stellt das Filmpflaster ebenfalls bei der Anwendung keine besonderen Anforderungen an die Haftung Folie/Stützmaterial. Mechanische Adhäsionskräfte reichen aus, so wie sie bei der Herstellung des Films durch Extrusion, Gießen, etc. der Folie auf dem Stützmaterial, erreicht werden, da die einzelnen Abschnitte des zumindest zweiteiligen oder teilbaren Abdeckmaterials nacheinander abgezogen werden können.
- Das Produkt erfordert keine zusätzlichen Anfasser und ist daher kostengünstig herzustellen
- Der Stützrahmen kann nach Applikation des Pflaster einfach abgezogen werden.

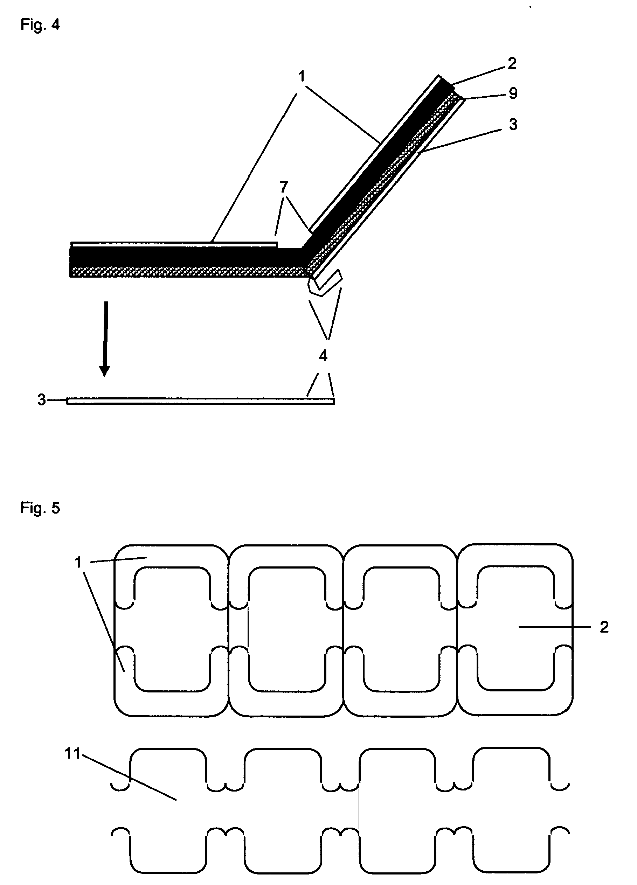

Um die maschinengängige Herstellung des Pflasters und ein Minimum an Verfahrensschritten zu gewährleisten, ist folgende Problemstellung zu berücksichtigen gewesen, die mittels der erfindungsgemäßen Ausgestaltung überraschend gelöst wurde.
Bei der Herstellung wird die Folie bahnförmig, ohne dass die einzelnen Filmpflaster separiert vorliegen, mit Klebmasse beschichtet. Üblicherweise ist die mit Klebstoff beschichtete Folie dann vollflächig mit dem Stützmaterial auf der nicht mit der Klebemasse beschichteten Seite beaufschlagt. Im nachfolgenden Schritt werden die Konturen des Stützrahmens aus dem Stützmaterial ohne Beschädigung der Folie ausgestanzt.
Üblichweise ist dies aber problematisch, da durch die erfindungsgemäß bevorzugte und ansonsten bei Pflastern auch übliche Überlappung des Abdeckmaterials ein Auflegen des Rahmenmaterials und anschließendes Ausstanzen des Rahmens im gleichen Herstellungsprozess nicht möglich ist. Beim Durchstanzen bzw. Ausstanzen des Rahmens würde sich ansonsten im erhabeneren Überlappungsbereich des Abdeckmaterials eine Beschädigung des Folienmaterials ergeben. Dies scheint auch ein Grund dafür zu sein, dass Folienpflaster aus dem Stand der Technik für die vollflächige Klebstoffabdeckung ein einstückiges Abdeckmaterial vorsehen.
Diesem Problem wird erfindungsgemäß begegnet indem die Stützrahmen zumindest zweigeteilt sind. D.h. bevorzugt im Bereich des Überlappungsbereiches des Abdeckmaterials findet keine Ausstanzung statt. Dies hat zur Folge, dass das übrig bleibende Stützmaterial (sog. Mittelteil), das vom Rahmen abgezogen werden muss, zusammenhängend über alle Filmpflaster einer Bahn ausgebildet ist. Es entsteht bei der Herstellung dann sozusagen eine durchgehende Kette an vom zurückbleibenden Stützrahmenteilen abgetrennten Mittelteilen. Die Mittelteile sind durchgehend, unununterbrochen und lassen sich in einem Stück von der Folie entfernen. Die abzuziehenden Mittelteile des Stützmaterials bilden somit eine ununterbrochene Bahn, wie es in Fig. 5 erläuternd skizziert ist.
Die Mittelteile, die die Unterbrechung des Stützrahmen bilden, sind demzufolge bevorzugt parallel zur Trennlinie, dem Überlappungsbereich des Abdeckmaterials ausgebildet ist. Die Kontur des Stützrahmens zum Mittelteil ist vorteilhaft dergestalt, dass sie parallel zur Trennlinie des Abdeckmaterials entsprechend einer Spline-Funktion ohne abrupte Übergänge gestaltet ist. Die Form der Mittelteile ist dabei von der Form der übrig bleibenden Rahmen vorgegeben (s. Fig. 5).

Die erfindungsgemäße Gestaltung der auf der Folie übrig bleibenden und damit unterbrochenen Stützrahmen ermöglicht somit erst die einfache maschinengängige Herstellung der Filmpflaster. Indem aus dem vollflächigen Stützmaterial die Stützrahmen so ausgestanzt werden, dass die abzuziehenden Mittelteile in einem Stück ununterbrochen entfernt werden können, ergibt sich der weiterer Vorteil einer einfachen Herstellweise.
Nach dem Abziehen bleibt ein stabilisierender, zumindest zweigeteilter unterbrochener Rahmen auf der Folie zurück, der bevorzugt im Überlappungsbereich des Abdeckmaterials unterbrochen ist. Vorteilhaft ist dabei eine trapezförmige Ausgestaltung der Rahmenschenkel. Es können auch mehr als zwei Stützrahmen und entsprechend mehrere Mittelteile ausgebildet sein.
Abschließend erfolgt die Separierung der einzelnen Pflaster. Nach der Herstellung der Pflaster kann die Einsiegelung in eine entsprechende Folie oder Papier erfolgen, um das Pflaster gegen äußere Verschmutzung oder Kontamination zu schützen.

Die Folie besteht vorzugsweise aus elastomeren Polymeren auf Polyurethan-, Polyester-, Polyamid- oder Polystyrolbasis, wie z.B. aromatischen und aliphatischen Polyester- oder Polyetherurethanen, Polyetherestern, Polyetheramiden oder Styrol-Block-Copolymeren, Blends aus diesen Polymeren oder auch mehrschichtigen coextrudierten Folien, bei denen mindestens eine Schicht aus einer dieser Polymere basiert. Auch Folien auf Polyolefinbasis, wie z.B. Polyethylen oder Polypropylen sind möglich. Vorteilhaft ist die Folie transparent gestaltet. Die Folie hat bevorzugt eine Dicke von 5 bis 100 µm, insbesondere bevorzugt im Bereich von 10 bis 60 µm, was als relativ dünn bezeichnet werden kann.

Eine bevorzugte Folie ist aus elastischen, thermoplastischen Polyurethan hergestellt. Sie ist etwa 25 bis 40 µm dick, transparent, weist eine Reißdehnung von über 450% und eine Wasserdampfdurchlässigkeit von mehr als 500 g/m² auf , bestimmt nach ASTM E 96.

Die Haftung der Folie auf dem Stützmaterial, die nur gering, d.h. zwischen 0,005 und 0,5 N/cm, zu sein braucht, wird vorzugsweise dadurch bewirkt, dass die Folie direkt auf dem Stützmaterial durch Extrusion, Gießen oder andere bekannte Methoden erzeugt wird. Das Stützmaterial kann ebenfalls aus einer Folie oder bevorzugt aus einem Papier bestehen.

Als Haftklebemassen für die Befestigung des Pflasters auf der Haut können alle handelsüblichen Haftklebemassen medizinischer Qualität eingesetzt werden. Beispielsweise können die Klebemassen auf Acrylatbasis hergestellt werden. Die Folie kann vollflächig beschichtet sein, ebenso ist jedoch eine partielle Beschichtung der Folie mit der Klebemasse möglich.

Die Klebemasse kann geschäumt sein, was eine bessere Haptik und eine verbesserte Luftund Wasserdampfdurchlässigkeit gewährleistet. Bevorzugt kommen als Klebemassen Heißschmelzklebemassen (hot-melts) zur Anwendung, wie sie beispielsweise in DE 4222334 oder DE 19620107 beschrieben sind.
Partieller Auftrag bedeutet hierbei ein Auftrag über die gesamte Fläche verteilt aber nicht ununterbrochen. Die nicht vollflächig, partiell aufgebrachte, bevorzugt auch geschäumte Klebeschicht kann dabei rasterförmig, vorzugsweise in Kalottenform durch Siebdruck, vorliegen oder auch in einem anderen Muster wie Gitter, Streifen, Zickzacklinien und beispielsweise auch durch Tiefdruck aufgebracht sein. Ferner kann sie beispielsweise auch aufgesprüht sein, was ein mehr oder weniger unregelmäßiges Auftragsbild ergibt.

Die Klebemasse kann gleichmäßig auf der Folie verteilt sein, sie kann aber auch funktionsgerecht für das Produkt über die Fläche unterschiedlich stark oder dicht aufgetragen sein. Dies bedeutet, dass an bestimmten beanspruchten Bereichen, z.B. den Rändern, mehr oder dichtere Klebstoffbereiche aufgetragen werden als an anderer Stelle.

Auf seiner klebend ausgerüsteten, später der Haut zugewandten Seite ist das erfindungsgemäße Folienpflaster über seine ganze Breite bis zum Gebrauch üblicherweise mit einem klebstoffabweisenden Abdeckmaterial, wie beispielsweise silikonisiertem Papier, abgedeckt. Dieses schützt die Selbstklebeschicht aus einer gut hautverträglichen Klebemasse und stabilisiert zusätzlich das ganze Produkt. Die Abdeckung ist in bekannter Weise vorzugsweise zweiteilig und überlappend ausgebildet. Die Abdeckung kann jedoch auch einteilig mit einer Perforation, als so genannter Splitliner, ausgebildet sein.

Der Anwender nimmt in gewohnter Weise das erfindungsgemäße Pflaster zur Hand und entfernt den einen Teil des Abdeckmaterials. Dies ist ihm im Gegensatz zum Filmpflaster nach EP 51935, EP 690706 oder dem auf dem Markt erhältlichen transparenten Wundverband Tegaderm® ohne Probleme möglich, da erfindungsgemäß das Abdeckmaterial zumindest in zwei Schritten vom Pflaster abgezogen wird.

Das Abdeckmaterial ist bevorzugt zumindest zweigeteilt ist. Die beiden Teile des Abdeckpapiers überlappen in einem bestimmten Bereich, dem Trenn- oder Überlappungsbereich. Dieser Bereich liegt bevorzugt mittig oder im äußeren Drittel bzw. Viertel der Gesamtfolienfläche eines Pflasters. An der Trennlinie des geteilten oder einteilig perforierten Abdeckmaterials kann der Anwender den abzuziehenden Teil des Abdeckmaterials problemlos und einfach anfassen und abziehen, wie er es von gängigen Pflasterapplikationen gewohnt ist. Anschließend lässt sich das erfindungsgemäße Pflaster mit der jetzt freigelegten klebstoffbeschichteten Seite auf die Haut auflegen. Vorteilhaft ist dabei, dass die Stützrahmen den Trenn- bzw. Überlappungsbereich des Abdeckmaterials freilassen und somit ein Knick des Folienpflasters genau in dem Bereich erfolgen kann, der den frei gelegten klebstoffbeschichtete Teil von dem abgedeckten Teil unterteilt (siehe Fig. 4).

Wie gewohnt kann dann das oder die weiteren Teile des Abdeckmaterials entfernt werden ohne das es zu einem Faltenwurf oder "Mit-sich-selbst-Verkleben" kommt, da die auf der oberen Seite der Folie aufgebrachten Stützrahmen dem Pflaster die nötige Stabilität verleihen. Die Stützrahmen können abschließend vom auf die Haut applizierten Pflaster entfernt werden.

Bevorzugt ist das erfindungsgemäße Pflaster rund, oval oder rechteckig ausgestaltet.
In Figur 1 ist das erfindungsgemäße Filmpflaster in dreidimensionaler Sicht dargestellt.
Figur 2 zeigt den prinzipiellen Schichtaufbau des Pflasters und in Figur 3 ist ein erfindungsgemäßes Pflaster in der Aufsicht gezeigt.
Fig. 4 zeigt das Pflaster von der Seite nach Entfernen des einen Abdeckteils und Applizieren auf die Haut.
Fig. 5 skizziert den Herstellschritt der Ausstanzung der Stützrahmen und Entfernung der Mittelteilbahn.

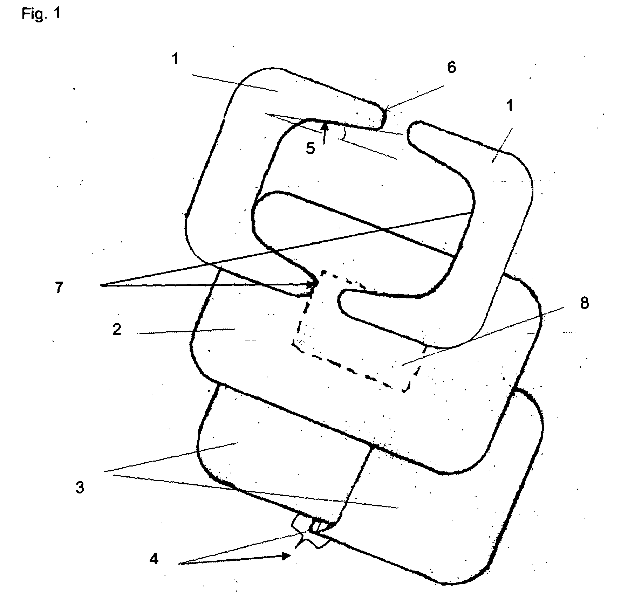

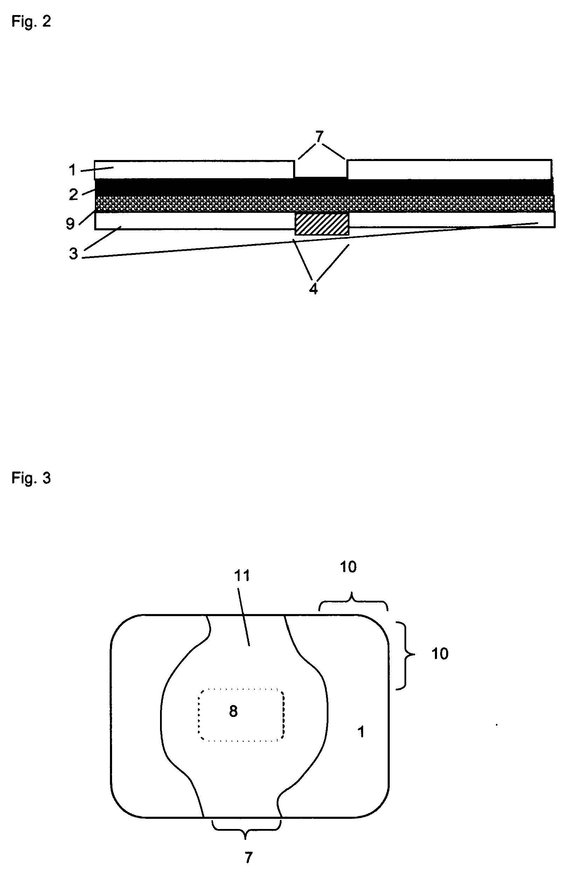

Fig. 1 zeigt das Filmpflaster umfassend eine relativ dünne Folie (2). Auf eine Seite der Folie, die Seite mit der das Pflaster in der Anwendung auf die Haut appliziert wird, wird eine Klebemasse (9) aufgebracht (s. Fig. 2). Die Klebemasse ist zumindest partiell, bevorzugt vollflächig auf der Folie (2) aufgebracht.
Auf der Klebemasse ist zum Schutz und zur besseren Handhabung ein zumindest zweigeteiltes Abdeckmaterial (3) aufgebracht. Dieses klassische Abdeckmaterial kann der Anwender vor dem Applizieren einfach ablösen. Das Abdeckmaterial ist bevorzugt zweigeteilt und bevorzugt überlappt an einer Trennlinie bzw. Überlappungsbereich (4). Diese Zweiteilung ist der Anwender von gewöhnlichen Pflastern, wie z.B. Hansaplast®, gewöhnt und erfordert keinerlei Umständlichkeiten bei der Applikation. Es ist sogar einhändig möglich, das erfindungsgemäße Pflaster zu applizieren. Da die Folie (2) relativ dünn ist, bevorzugt eine Dicke von 10 - 60 µm aufweist, sind zur Stabilisierung Stützrahmen (1) vorgesehen, die bevorzugt im Randbereich (10) der Folie aufgebracht sind (siehe Fig. 3). Die Stützrahmen (1) sind auf der Seite der Folie (2) aufgebracht, die nicht die Klebemasse (9) trägt, definiert als die der Klebemasse abgewandten Seite.

Die Kontur der Stützrahmen ist so gestaltet, dass das Abziehen des Mittelteils des Stützmaterials (11) bei der Herstellung der Pflaster einfach möglich ist. In Richtung der Trennlinie (4) des Abdeckmaterials ist die Kontur (7) der Stützrahmen entsprechend einer Spline - Funktion ohne abrupte Übergänge gestaltet. Der Unterbrechungsbereich des Rahmens kann vorteilhaft gleich oder größer dem Überlappungsbereich der Abdeckfolie (4) sein (Fig. 2).
Die bevorzugt trapezförmige Gestaltung ist durch einen entsprechenden Winkel (5) herstellbar, bevorzugt mit einer Abrundung des Stützrahmens (6).

Fig. 5 zeigt die noch nicht separierten Filmpflaster bei denen das Mittelteil (11) einstückig abgezogen ist. Übrigbleiben die Filmpflaster mit Stützrahmen (1) auf der Folie (2).

Die Stützrahmen (1) können nach Applikation des Pflaster auf der Haut einfach abgezogen werden. In der Anwendung als Wundpflaster enthält das erfindungsgemäße Filmpflaster eine übliche Wundauflage (8) z.B. aus Vliesstoff oder Gewirke aber auch auf Hydrogel oder Hydokolloidbasis, die bevorzugt mittig auf der Folie aufgebracht ist. Das erfindungsgemäße Pflaster kann allerdings auch ohne Wundauflage ausgeführt sein.

Das Produkt kann nach Standard-Verfahren verpackt und sterilisiert werden.

## Patentansprüche

1. Filmpflaster umfassend
a. eine Folie (2) von bis zu 100 µm Dicke, bestehend aus mindestens einem wasserdampfdurch- und wasserundurchlässigem Polymer,
b. eine Klebemasse (9), die zumindest partiell eine Seite der Folie (2) beschichtet,
c. ein zumindest zweigeteiltes oder teilbares Abdeckmaterial (3), das auf der Klebemassen (9) aufgebracht ist,
d. einen zumindest zweigeteilten Stützrahmen (1), der auf der der Klebemasse abgewandten Seite auf der Folie (2) angebracht ist, und
e. ein Trenn- bzw. Überlappungsbereich (4) des Abdeckmaterials (3)
**dadurch gekennzeichnet, dass**
der Stützrahmen (1) unterbrochen ist und den Trenn- bzw Überlappungsbereich (4) des Abdeckmaterials (3) frei lässt.

2. Filmpflaster nach Anspruch 1, **dadurch gekennzeichnet, dass** die Unterbrechung des Stützrahmen (1) parallel zur Trennlinie (4) des Abdeckmaterials ausgebildet ist.

3. Filmpflaster nach Anspruch 1 oder 2, **dadurch gekennzeichnet, dass** die Kontur (7) des Stützrahmen (1) zum Mittelteil (11) parallel zur Trennlinie (4) des Abdeckmaterials entsprechend einer Spline-Funktion ohne abrupte Übergänge gestaltet ist.

4. Filmpflaster nach einem der vorstehenden Ansprüche, **dadurch gekennzeichnet, dass** die Klebemasse (9) vollflächig auf der Folie (2) aufgebracht ist.

5. Filmpflaster nach einem der vorstehenden Ansprüche, **dadurch gekennzeichnet, dass** die Folie (2) transparent ist.

6. Filmpflaster nach einem der vorstehenden Ansprüche, **dadurch gekennzeichnet, dass** die Folie (2) eine Dicke von 10 bis 60 µm aufweist.

7. Filmpflaster nach einem der vorstehenden Ansprüche, **dadurch gekennzeichnet, dass** die Folie (2) aufgebaut ist aus einem oder mehreren Polymer gewählt aus der Gruppe der elastomeren Polymeren auf Polyurethan-, Polyester-, Polyamid- oder Polystyrolbasis, bevorzugt aromatische und aliphatische Polyester- oder Polyetherurethane, Polyetherester, Polyetheramide oder Styrol-Block-Copolymere oder Blends aus diesen oder mit anderen Polymeren oder mehrschichtigen Folien, bei denen mindestens eine Schicht auf einem dieser Polymere basiert.

8. Filmpflaster nach einem der vorstehenden Ansprüche, **dadurch gekennzeichnet, dass** die Klebemasse (9) eine Acrylatmasse ist,

9. Filmpflaster nach einem der vorstehenden Ansprüche, **dadurch gekennzeichnet, dass** auf der Klebemasse (9) eine Wundauflage (8) aufgebracht ist.

## Claims

1. Film-backed plaster comprising
a. a film (2) of up to 100 µm in thickness, composed of at least one water vapour-permeable and water-impermeable polymer,
b. an adhesive (9) which at least partially coats one side of the film (2),
c. a liner material (3) which is divisible or divided into at least two and is applied to the adhesive (9),
d. a support frame (1) which is divided into at least two and is mounted on the side of the film (2) that faces away from the adhesive, and
e. a separating region or overlap region (4) of the liner material (3),
**characterized in that**
the support frame (1) is interrupted and exposes the separating region or overlap region (4) of the liner material (3).

2. Film-backed plaster according to Claim 1, **characterized in that** the interruption of the support frame (1) is formed parallel to the separating line (4) of the liner material.

3. Film-backed plaster according to Claim 1 or 2, **characterized in that** the contour (7) of the support frame (1) to the middle part (11) parallel to the separating line (4) of the liner material is formed in accordance with a spline function without abrupt transitions.

4. Film-backed plaster according to any one of the preceding claims, **characterized in that** the adhesive (9) is applied over the full area of the film (2).

5. Film-backed plaster according to any one of the preceding claims, **characterized in that** the film (2) is transparent.

6. Film-backed plaster according to any one of the preceding claims, **characterized in that** the film (2) has a thickness of 10 to 60 µm.

7. Film-backed plaster according to any one of the preceding claims, **characterized in that** the film (2) is composed of one or more polymers selected from the group of elastomeric polymers based on polyurethane, polyester, polyamide or polystyrene, preferably aromatic and aliphatic polyester- or polyetherurethanes, polyetheresters, polyether-amides or styrene block copolymers or blends of these or with other polymers or multi-layer films in which at least one layer is based on one of these polymers.

8. Film-backed plaster according to any one of the preceding claims, **characterized in that** the adhesive (9) is an acrylate composition.

9. Film-backed plaster according to any one of the preceding claims, **characterized in that** a wound contact material (8) is applied on the adhesive (9).

## Revendications

1. Pansement en pellicule, comprenant :
a. une feuille (2) d'une épaisseur de jusque 100 µm, constituée d'au moins un polymère imperméable à la vapeur d'eau et à l'eau,
b. une pâte adhésive (9) qui recouvre au moins une partie d'un côté de la feuille (2),
c. un matériau de recouvrement (3) divisé ou divisible en au moins deux parties et appliqué sur la pâte adhésive (9),
d. un encadrement de soutien (1) divisé en au moins deux parties et appliqué sur le côté de la feuille (2) non tournée vers la pâte adhésive et
e. une zone (4) de séparation ou de superposition du matériau de recouvrement (3),
**caractérisé en ce que**
l'encadrement de soutien (1) est interrompu et libère la zone (4) de séparation ou de superposition du matériau de recouvrement (3).

2. Pansement en pellicule selon la revendication 1, **caractérisé en ce que** l'interruption de l'encadrement de soutien (1) est parallèle à la ligne de séparation (4) du matériau de recouvrement.

3. Pansement en pellicule selon les revendications 1 ou 2, **caractérisé en ce que** le contour (7) de l'encadrement de soutien (1) autour de la partie centrale (11) est parallèle à la ligne de séparation (4) du matériau de recouvrement en suivant une fonction en cannelures sans transitions abruptes.

4. Pansement en pellicule selon l'une des revendications précédentes, **caractérisé en ce que** la pâte adhésive (9) est appliquée sur toute la surface de la feuille (2).

5. Pansement en pellicule selon l'une des revendications précédentes, **caractérisé en ce que** la feuille (2) est transparente.

6. Pansement en pellicule selon l'une des revendications précédentes, **caractérisé en ce que** la feuille (2) a une épaisseur de 10 à 60 µm.

7. Pansement en pellicule selon l'une des revendications précédentes, **caractérisé en ce que** la feuille (2) est constituée d'un ou plusieurs polymères sélectionnés dans l'ensemble constitué des polymères élastomères à base de polyuréthane, de polyester, de polyamide ou de polystyrène, de préférence de polyesteruréthanes ou de polyétheruréthanes aromatiques ou aliphatiques, de polyétherester, de polyétheramide ou de copolymères séquencés de styrène, et de leurs mélanges entre eux ou avec d'autres polymères ou feuilles stratifiées dont au moins une couche est basée sur l'un de ces polymères.

8. Pansement en pellicule selon l'une des revendications précédentes, **caractérisé en ce que** la pâte adhésive (9) est une pâte d'acrylate.

9. Pansement en pellicule selon l'une des revendications précédentes, **caractérisé en ce qu'**une couche (8) de recouvrement de la blessure est appliquée sur la pâte adhésive (9).
